(19) Europäisches Patentamt
European Patent Office
Office européen des brevets

(11) **EP 4 094 801 A1**

(12) **EUROPEAN PATENT APPLICATION**
published in accordance with Art. 153(4) EPC

(43) Date of publication:
**30.11.2022 Bulletin 2022/48**

(21) Application number: **21744060.1**

(22) Date of filing: **21.01.2021**

(51) International Patent Classification (IPC):
**A61N 1/20** (2006.01)

(52) Cooperative Patent Classification (CPC):
**A61N 1/04; A61N 1/20**

(86) International application number:
**PCT/JP2021/002059**

(87) International publication number:
**WO 2021/149769 (29.07.2021 Gazette 2021/30)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **21.01.2020 JP 2020007538
02.06.2020 JP 2020095997**

(71) Applicant: **Iongear Co., Ltd.
Chiba-shi, Chiba, 260-0013 (JP)**

(72) Inventors:
• **OGAMA, Kenji
Chiba-shi, Chiba 267-0066 (JP)**
• **OGAMA, Yusuke
Chiba-shi, Chiba 267-0066 (JP)**
• **OGAMA, Kohei
Chiba-shi, Chiba 267-0066 (JP)**
• **OGAMA, Keisuke
Chiba-shi, Chiba 267-0066 (JP)**

(74) Representative: **Glawe, Delfs, Moll
Partnerschaft mbB von
Patent- und Rechtsanwälten
Postfach 13 03 91
20103 Hamburg (DE)**

(54) **BIOGALVANIC BATTERY MEDICAL APPLIANCE**

(57) Provided is a biogalvanic battery therapeutic appliance that can effectively pass electric current even at a negative electrode with an extended length and a high resistance value, achieving excellent current stimulation effect with comfortable touch. This biogalvanic battery medical appliance is configured such that a negative electrode component (11) and a positive electrode component (12) are connected to a conductive member (13) and are brought into contact with a skin forming an energized circuit in the skin. The conductive member (13) connects the negative electrode component (11) and the positive electrode component (12) between a surface of the negative electrode component (11) and a surface of the positive electrode component (12) on the opposite side from a skin contact surface. The conductive member (13) and the positive electrode component (12) are both made of the same carbon material.

FIG. 2A

FIG. 2B

EP 4 094 801 A1

# FIG. 2A

11    12
(Skin-contacting surface)

(Surface side that is opposite
to the skin-contacting surface)
13

# FIG. 2B

11    12
(Skin-contacting surface)

(Surface side that is opposite
to the skin-contacting surface)
13

**Description**

TECHNICAL FIELD

This invention relates to a biogalvanic battery therapeutic appliance, and specifically relates to a biogalvanic battery therapeutic appliance that is used upon having been brought into contact with the skin and that treats a target site by the electric stimulation of subcutaneous tissue with a weak direct-current electromotive force applied thereto.

BACKGROUND ART

In recent years, the number of patients suffering from chronic stiff shoulders and lower-back pain has been increasing, and to date many poultices, indirect moxa treatments, metal pellets, magnetic therapeutic appliances, low-frequency therapeutic devices, etc., have been marketed as home-use therapeutic appliances. These therapeutic appliances have the effect of promoting blood circulation in the affected area by means of various principles and purifying locally accumulated waste products.

The inventors have previously proposed biogalvanic battery therapeutic appliances that heal muscle and nerve fatigue by electric stimulation (see Patent Documents 1 and 2). Said biogalvanic battery therapeutic appliances each form a biogalvanic battery and apply a direct current when in contact with the skin, and have been demonstrated to be excellent therapeutic appliances having a therapeutic effect as home-use therapeutic appliances.

However, these home-use therapeutic appliances have the following problem to be improved on.

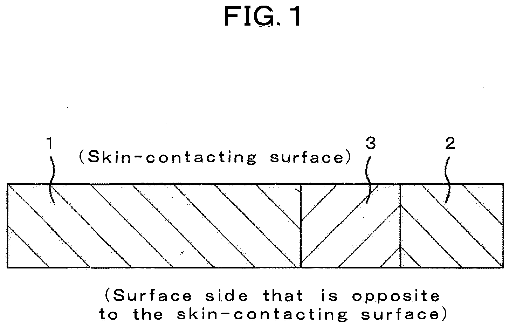

Hereinafter, the configuration of a previously proposed biogalvanic battery therapeutic appliance is schematically shown in FIG. 1, and a problem to be improved on shall be described. In FIG. 1, reference numeral 1 indicates a negative electrode (e.g., a metal powder mixed in a binder), 2 indicates a positive electrode (e.g., either a noble metal or a noble metal mixed in a binder), 3 indicates an electroconductive member (e.g., carbon) composed of a substance that does not have an ionization tendency, the electroconductive member 3 being interposed between the negative electrode 1 and the positive electrode 2.

In this type of biogalvanic battery therapeutic appliance, the negative electrode 1 is formed long so that a large amount of current generated by ionization of the negative electrode component flows to the positive electrode. However, the negative electrode, which has a configuration in which metal powder such as zinc is mixed in the binder, has high resistance, the electrical resistance value (volume resistance value) thereof being at minimum 1 to 2 MΩ·cm; therefore, the distance to reach the electroconductive member is increased, and it is substantially difficult for the current to reach the positive electrode. As a result, it has been difficult to obtain an efficient biogalvanic battery therapeutic appliance.

PRIOR ART

Patent Documents

Patent Document 1: Japanese Patent Publication No. 6168639
Patent Document 2: Japanese Patent Publication No. 6153259

DISCLOSURE OF THE INVENTION

Problems the Invention Is Intended to Solve

The inventors have conducted research in order to solve this problem, and in having focused on the fact that the thickness between a surface where the negative electrode comes into contact with a living subject and a surface opposing said contact surface is extremely thin, configured this type of biogalvanic battery therapeutic appliance such that an electroconductive member is disposed on the surface of the negative electrode opposing the surface that comes into contact with the living subject, and a current is transmitted to the positive electrode through the electroconductive member so disposed. Ions thereby reach the electroconductive member not in the length direction of the negative electrode constituent member but in the thickness direction of the negative electrode constituent member, in which the distance is short. As a result, the distance over which ions pass through the negative electrode can be kept extremely short; therefore, the inventors perfected the invention upon discovering that the therapeutic effect of this type of biogalvanic battery therapeutic appliance can be effectively exhibited even if the electrical resistance value of the negative electrode constituent member is high.

The present invention was arrived at based on the above findings, providing a biogalvanic battery therapeutic appliance in which, even if the negative electrode constituent member having a high electrical resistance value is formed long and the distance from the negative electrode to the positive electrode is large, an electroconductive member is disposed and formed on a front-surface side (i.e., the surface side that does not come into contact with the skin) of the negative electrode constituent member that is opposite to the skin-contacting surface, thereby shortening the distance needed for the ionized negative electrode constituent substance to reach the electroconductive member, whereby ions can efficiently reach the positive electrode even if the distance from the negative electrode to the positive electrode constituent member is large.

Means for Solving the Problems

The present invention has been configured as follows in order to solve the problems described above.

(1) A biogalvanic battery therapeutic appliance comprising a negative electrode constituent member, a positive electrode constituent member, and an electroconductive member connected and disposed between the negative electrode constituent member and the positive electrode constituent member, electric circuits being formed between a living subject and the negative electrode constituent member and between the living subject and the positive electrode constituent member by bringing the negative electrode constituent member and the positive electrode constituent member into contact with the living subject, wherein

the negative electrode constituent member and the positive electrode constituent member each having a skin-contacting surface that comes into contact with the skin and an opposing surface formed on a surface that opposes the skin-contacting surface,
the negative electrode constituent member and the positive electrode constituent member being electrically connected by bridging and connecting the electroconductive member between the opposing surface of the negative electrode constituent member and the opposing surface of the positive electrode constituent member, and
the biogalvanic battery therapeutic appliance is characterized in that the electroconductive member and the positive electrode constituent member are constituted of same carbon material.

(2) The biogalvanic battery therapeutic appliance according to (1), wherein the electroconductive member bridged and connected between the opposing surface of the negative electrode constituent member and the opposing surface of the positive electrode constituent member is disposed on the surface side opposite to the skin-contacting surface of the negative electrode constituent member, extending to the positive electrode constituent member from a starting point that is the location where the distance from the positive electrode constituent member is the longest.
(3) The biogalvanic battery therapeutic appliance according to (1), wherein the electroconductive member is formed as a coating on at least the entire surface of the skin-contacting surface side of the negative electrode constituent member.
(4) The biogalvanic battery therapeutic appliance according to any of (1) to (3), wherein the negative electrode constituent member and the positive electrode constituent member are disposed separate from and opposing each other.
(5) The biogalvanic battery therapeutic appliance according to any of (1) to (3), wherein the negative electrode constituent member and the positive electrode constituent member are disposed in contact with each other.
(6) The biogalvanic battery therapeutic appliance according to any of (1) to (5), wherein the electroconductive member has an electrically insulating layer formed as a coating on the top surface of the surface opposite to the skin elsewhere beside the area that comes into contact with the positive electrode constituent member and the negative electrode constituent member, and contact between the electroconductive member and the skin is blocked by the electrically insulating layer.
(7) The biogalvanic battery therapeutic appliance according to any of (1) to (6), wherein the negative electrode constituent member has a length of 10 to 200 mm, a thickness of 150 $\mu$m or less, and a "negative electrode constituent member length / negative electrode constituent member thickness" ratio of 1 or greater.
(8) The biogalvanic battery therapeutic appliance according to (7), wherein the negative electrode constituent member has a "negative electrode constituent member length / negative electrode constituent member thickness" ratio of 100 or greater.

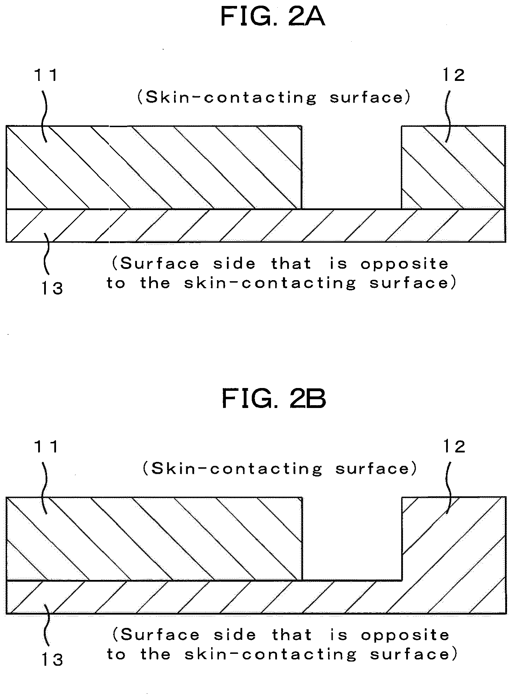

In the present invention, the "thickness of the negative electrode constituent member" is, referring to FIG. 2, the vertical thickness of a negative electrode constituent member 11, and in the biogalvanic battery therapeutic appliance according to the present invention, current flows in the "thickness direction of the negative electrode constituent member."
In addition, the "length of the negative electrode constituent member" is, referring to FIG. 2, the lateral length of the negative electrode constituent member 1, and in a prior-art biogalvanic battery therapeutic appliance (configured such that an electroconductive member is interposed between a negative electrode and a positive electrode) such as is shown in FIG. 1, current flows in the direction of the "length of the negative electrode constituent member."

The term "substantially L-shaped" refers to a configuration including a base piece and a rising piece rising from one end of the base piece, the orientation of the configuration being irrelevant. A biogalvanic battery therapeutic appliance comprising other parts as well as parts constituting a substantial L shape is also included.

By negative electrode constituent member is meant a member that includes a component constituting a negative electrode and that functions as a negative electrode, and by positive electrode constituent member is meant a member that includes a component constituting a positive electrode and that functions as a positive electrode.

First, a basic summary of the present invention shall be described on the basis of the schematic drawing of FIG. 2.

In FIG. 2A, 11 indicates a negative electrode constituent member, 12 indicates a positive electrode constituent member, and 13 indicates an electroconductive member electrically connecting the negative electrode constituent member and the positive electrode constituent member. In FIG. 2A, the upper surface indicates the skin-contacting surface side, and the lower surface indicates the surface opposite to the skin-contacting surface side (referred to below as the opposing surface).

The negative electrode constituent member 11 and the positive electrode constituent member 12 are disposed apart from each other, and the electroconductive member 13 is disposed bridging the opposing-surface sides of the negative electrode constituent member 11 and the positive electrode constituent member 12.

In FIG. 2, the negative electrode constituent member 11 and the positive electrode constituent member 12 are disposed apart from each other, but in the present invention, the negative electrode constituent member 11 and the positive electrode constituent member 12 can also be in contact with each other. This is because in this type of biogalvanic battery therapeutic appliance, for example, the electrical resistance of the negative electrode constituent member 11 is about 1 to 2 M$\Omega$ , and the electrical resistance of the electroconductive member 13 is about 1 to 50$\Omega$ .

Therefore, the electrical resistance value of the negative electrode constituent member 11 is high even if the negative electrode constituent member 11 and the positive electrode constituent member 12 are brought into contact, and therefore ions generated in the negative electrode constituent member 11 essentially move from the negative electrode constituent member 11 to the positive electrode constituent member 12 through the electroconductive member 13 (which has a low electrical resistance value).

By negative electrode constituent member 11 is meant a component in which the negative electrode component included in this member has a greater ionization tendency than the positive electrode component included in the positive electrode constituent member 12. There are no particular limitations as to the negative electrode component constituting the negative electrode; zinc is an example of a highly practical material.

By positive electrode constituent member 12 is means a member in which the positive electrode component included in this member has a lesser ionization tendency than the negative electrode component included in the negative electrode constituent member 11. There are no particular limitations as to the positive electrode component constituting the positive electrode; noble metals in particular are examples of highly practical materials. The term "noble metal" according to the invention includes, *inter alia,* positive resins, and components of which at least the front-surface area is plated or otherwise covered with a noble metal. For example, silver-coated copper powder and the like also fall under the noble metal according to the present invention.

Because the positive electrode constituent member 12 can also be formed of carbon, it is also possible for the positive electrode constituent member 12 to be of the same substance as the electroconductive member 13, as shall be described hereinafter (refer to FIG. 2B).

Next, the electroconductive member 13 means "a member made of an electroconductive material that does not constitute a positive or negative electrode of a biogalvanic battery, or a member containing this material," and furthermore "a member that is made of an electroconductive material that is capable of constituting a positive or negative electrode of a biogalvanic battery, but that essentially does not come into contact with the skin and therefore does not constitute a positive or negative electrode of a biogalvanic battery."

There are no particular limitations as to the material of the electroconductive member 13; examples of highly practical materials include, particularly, carbon, electroconductive polymers, and the like. In the case of carbon, the electroconductive member is normally formed using, for example, carbon paint, a binder, printing, or the like. In addition, if a gel-form electroconductive polymer is applied, the polymer will inherently have an adhesive effect, and it is not necessary to combine the polymer with an adhesive, a binder, a filler, or the like.

In the present invention, "electroconductive" in the electroconductive member means that the electrical resistivity $\rho$ [$\Omega$ m] in the electrical resistance R [$\Omega$ ] determined in the following formula

$$R = \rho \cdot L/A \text{ (R: electrical resistance, L: length [m], A:}$$

$$\text{cross-sectional area } [m^2])$$

is preferably 1 $\Omega$ m or less, more preferably $10^{-2}$ $\Omega$ m or less, and particularly preferably $10^{-4}$ $\Omega$ m or less, and particularly if the electroconductive member contains a metal, the electrical resistivity can be $10^{-5}$ to $10^{-8}$ $\Omega$ m. Incidentally, the electrical resistivity $\rho$ of human skin is approximately $5.0 \times 10^5$ $\Omega$ m.

Though already stated previously, in the present invention, the positive electrode 12 can be constituted of the same material as the carbon constituting the electroconductive member 13, as shown in FIG. 2B. As such, in FIG. 2B, the positive electrode constituent member 12 and the electroconductive member 13 share the same hatching despite different reference numerals being used in order to differentiate between the two.

In the present specification, "skin" means the skin, mucous membrane, etc., of a living subject (human body, animal, etc.) in a broad sense, and means an area to which the biogalvanic battery therapeutic appliance according to the present invention can be attached.

Effects of the Invention

In the biogalvanic battery therapeutic appliance according to the present invention, the electroconductive member forming the path flowing from the negative electrode constituent member to the positive electrode constituent member is formed on the front-surface sides (referred to as the "opposing surfaces" in the present invention) opposing the skin-contacting surfaces (the surfaces where the negative electrode constituent member and the positive electrode constituent member come into contact with the skin). Therefore, even if the negative electrode constituent member is formed long using a material having a high electrical resistivity, electrons (current) generated by an ionized negative electrode constituent substance can pass through the electroconductive member formed on the opposing surfaces. Therefore, the distance needed for electrons (current) to reach the electroconductive member can be made extremely small. As a result, electrons (current) can efficiently reach the positive electrode through the electroconductive member even in a biogalvanic battery therapeutic appliance in which the negative electrode is formed long and the distance from the negative electrode to the positive electrode is substantially large.

More specifically, although this type of biogalvanic battery therapeutic appliance differs slightly depending on factors such as the method of use, the negative electrode constituent member is formed extremely thin (e.g., 150 $\mu$m or less, 100 $\mu$m or less, 50 $\mu$m or less, 10 $\mu$m or less, or preferably 5 to 10 $\mu$m), and the distance over which the ionized negative electrode constituent substance reaches the electroconductive member is an extremely small distance corresponding to the thickness of the negative electrode constituent member at the maximum, and is too small to be comparable with the length of the negative electrode constituent member. As a result, the biogalvanic battery therapeutic appliance will substantially not be adversely affected by the negative electrode constituent member, which has high electrical resistivity.

BRIEF DESCRIPTION OF THE DRAWINGS

FIG. 1 is a schematic drawing of a biogalvanic battery therapeutic appliance of the prior art;

FIG. 2 includes schematic drawings of the biogalvanic battery therapeutic appliance of the present invention, in which FIG. 2A shows an example in which the positive electrode member and the electroconductive member are different substances and FIG. 2B shows an example in which the positive electrode member and the electroconductive member are the same substance (carbon);

FIG. 3 is a schematic drawing of a biogalvanic battery therapeutic appliance of a first example according to the present invention, and the steps for manufacturing the appliance;

FIG. 4A is an explanatory drawing of a biogalvanic battery therapeutic appliance of a second example according to the present invention, FIG. 4B is an explanatory drawing of a biogalvanic battery therapeutic appliance of a third example, and 4C is an explanatory drawing of an ion current band of the third example;

FIG. 5 is an exploded perspective view of the biogalvanic battery therapeutic appliance of the second example according to the present invention;

FIG. 6 is an explanatory drawing of the steps of manufacturing a different H-shaped biogalvanic battery therapeutic appliance according to the present invention; and

FIG. 7 is an explanatory drawing of the steps of manufacturing a different circular biogalvanic battery therapeutic appliance according to the present invention.

Best Mode For Carrying Out The Invention

Examples of the present invention shall be described below.

In the biogalvanic battery therapeutic appliances of these examples, the thickness of the printed carbon-layer film is 10 $\mu$m as measured in cross section, the resistance value (top surface resistance value) of the printed carbon-layer film is 40Q , the thickness of the printed zinc-layer film is 20 $\mu$m as measured in cross section, and the weight or weight ratio of zinc in the coating of zinc-blended silicon is calculated to be 1.349 g of zinc and 0.193 g of Si ink.

(First example)

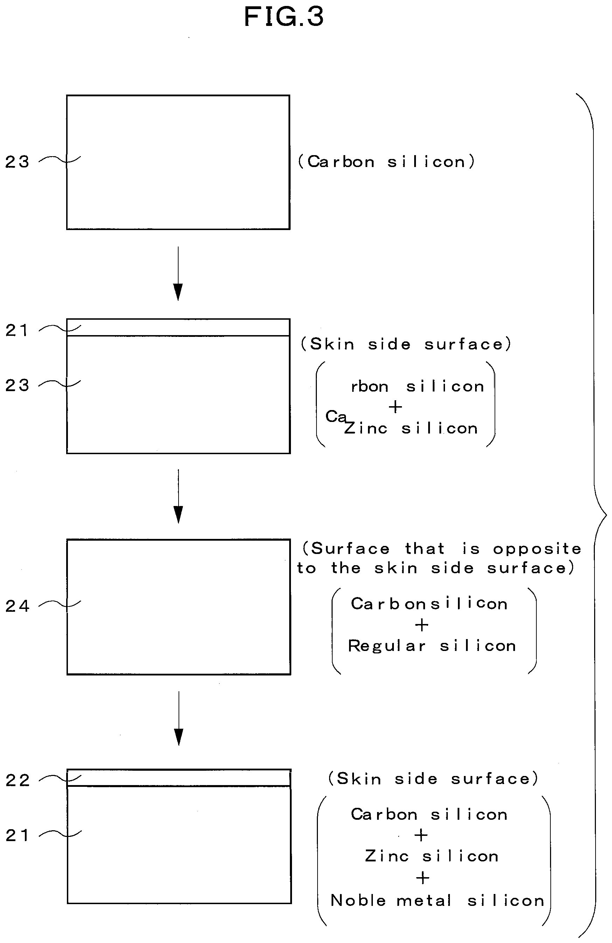

FIG. 3 is a schematic explanatory drawing of the steps of manufacturing a sheet-form biogalvanic battery therapeutic appliance, which is the first example.

(1) First, a carbon silicon sheet 23 (electroconductive member) is prepared as shown in the uppermost diagram in FIG. 3.

(2) Next, zinc silicon 21 (negative electrode constituent member) is printed from above the carbon-silicon sheet 23 on the surface side that will come into contact with the skin, as shown in the second diagram from the top of FIG. 3 (the top surface side of this diagram is the surface side that will come into contact with the skin). In this example, the carbon silicon sheet 23 (electroconductive member) is otherwise left exposed.

(3) Next, regular silicon 24 (insulating layer) is applied over and thereby printed on the entire surface of the carbon silicon sheet 23 (electroconductive member) that is opposite to the surface that will come into contact with the skin, as shown in the third diagram from the top of FIG. 3 (the top surface side of this diagram is the surface side opposite to the surface that will come into contact with the skin).

(4) Then, noble metal silicon 22 (positive electrode constituent member) is applied over and thereby printed on the surface (the upper side of the second diagram in FIG. 3) where the carbon silicon sheet 23 (electroconductive member) is exposed, as shown in the lowest diagram of FIG. 3 (the top surface side of this diagram is the surface side that will come into contact with the skin).

In the biogalvanic battery therapeutic appliance thus configured, the surface on which the zinc silicon 21 (negative electrode constituent member) and the noble metal silicon 22 (positive electrode constituent member) are formed (the top surface side in the lowest diagram in FIG. 3) is brought into contact with the skin, so that ions from the negative electrode constituent substance efficiently flow to the positive electrode constituent member through the low-electrical-resistance carbon silicon sheet layer (electroconductive member) on the surface opposite to the surface that will come into contact with the skin). As a result, current effectively flows to the skin with which the positive electrode constituent substance and the negative electrode constituent substance of the biogalvanic battery therapeutic appliance come into contact, and the purpose of the biogalvanic battery therapeutic appliance is efficiently achieved.

In this example, the zinc silicon 21 (negative electrode constituent member) and the noble metal silicon 22 (positive electrode constituent member) are in direct contact with each other, but because the zinc silicon 21 (negative electrode constituent member) has a high electrical resistance value, in essence, ions from the negative electrode constituent substance flow to the positive electrode constituent member 22 through the low-electrical-resistance carbon silicon sheet 23 (electroconductive member) on the surface opposite to the surface that comes into contact with the skin.

(Second example)

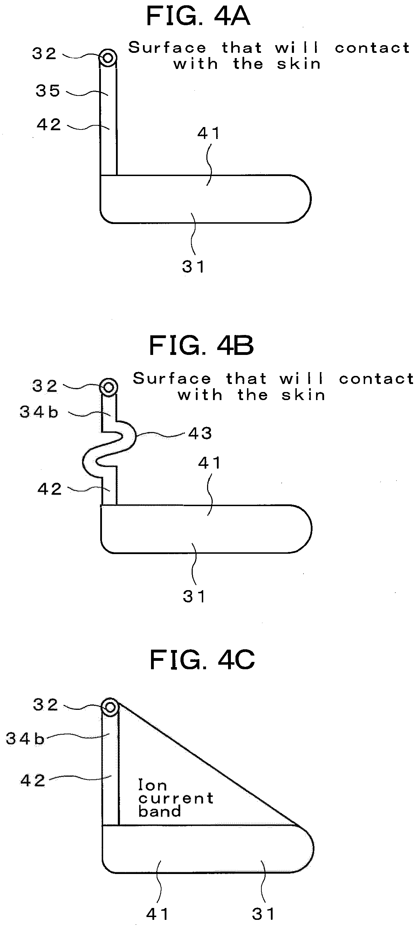

FIG. 4A shows an example of a substantially L-shaped biogalvanic battery therapeutic appliance comprising a base piece 41 and a rising piece 42 rising from one end of the base piece. This biogalvanic battery therapeutic appliance is effective for the face and especially, *inter alia,* the outer corners of the eyes where there is little muscle movement, and the front surface side in the diagram shows the skin-contacting surface. This biogalvanic battery therapeutic appliance comprises the wide base piece 41 and the narrow rising piece 42 rising from one end of the base piece 41. In FIG. 4, reference symbol 31 indicates a negative electrode constituent member provided to the base piece 41, reference symbol 32 indicates a button-form positive electrode constituent member provided to the tip of the rising piece 42, and reference symbol 35 indicates an insulating layer (first insulating layer).

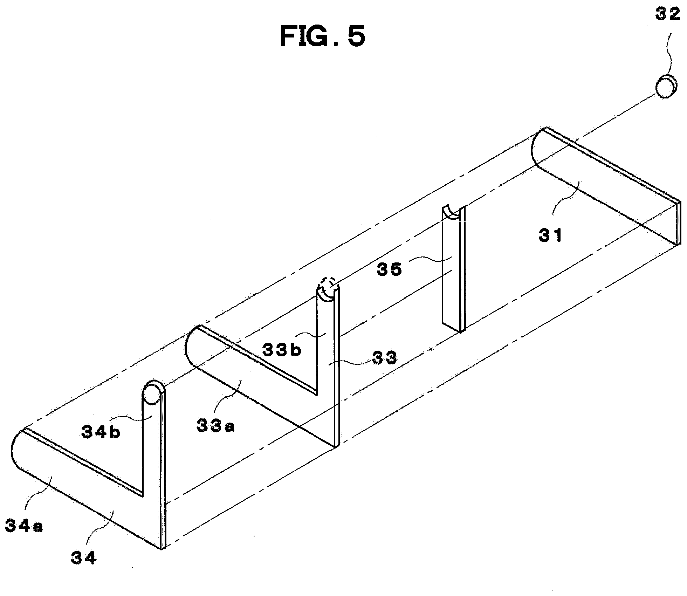

FIG. 5 shows an exploded perspective view of the biogalvanic battery therapeutic appliance of FIG. 4A. In FIG. 5, reference symbol 33 indicates a substantially L-shaped electroconductive member, reference symbol 33a indicates a base piece of the electroconductive member, and reference symbol 33b indicates a rising piece of the electroconductive member. The left side of the electroconductive member 33 in FIG. 5 (the top surface side opposite to the skin-contacting surface in the biogalvanic battery therapeutic appliance) is provided with a substantially L-shaped second insulating layer 34 (base piece 34a, rising piece 34b) that covers the electroconductive member 33.

The right side of the electroconductive member 33 in FIG. 5 (the skin-contacting surface side in the biogalvanic battery therapeutic appliance) is provided with the first insulating layer 35, and the rising piece 33b of the substantially L-shaped electroconductive member 33 is covered by the first insulating layer 35. However, the first insulating layer 35 is not covered by the thick part of the rising piece 33b of the electroconductive member 33 or by the tip of the thick part (the location where the positive electrode constituent member 32 is attached in this example).

Furthermore, the negative electrode constituent member 31 is provided on the right side of the electroconductive member 33 in FIG. 5 (the skin-contacting surface side in the biogalvanic battery therapeutic appliance), and the negative electrode constituent member 31 covers the skin-contacting surface side of the base piece 33a of the substantially L-shaped electroconductive member 33.

In addition, the button-form positive electrode constituent member 32 is provided at the tip of the rising piece 33b of the electroconductive member 33 (see FIG. 4A).

In the biogalvanic battery therapeutic appliance thus configured, when the negative electrode constituent member 31 and the positive electrode constituent member 32 are brought into contact with the covering, electrons generated from the ionized negative electrode component of the negative electrode constituent member 31 pass through the electroconductive member 33 to enter the positive electrode constituent member 32, and the desired current passes through the skin. Therefore, even if the electrical resistance value of the negative electrode constituent member 31 is high, the current flows through the thin negative electrode constituent member 31 from the electroconductive member 33 to the positive electrode constituent member 32, and thus the problem of the negative electrode constituent member 31 having a high electrical resistance value of can be solved and current can be caused to flow efficiently.

(Third example)

FIG. 4B shows another example of a substantially L-shaped biogalvanic battery therapeutic appliance, and shows a biogalvanic battery therapeutic appliance that is effective for the face and especially, *inter alia,* the smile lines where there is much muscle movement.

The basic configuration and operative effects of this biogalvanic battery therapeutic appliance are the same as those of the biogalvanic battery therapeutic appliance of FIGS. 4A and 5, but the difference is that a spring part 43 is formed partway along the rising piece 42, enabling the rising piece 42 to elastically deform following muscle movement. Due to the appliance being thus configured, even if the corners of the mouth are moved, the biogalvanic battery therapeutic appliance elastically deforms correspondingly, and as a result, the function of the invention can be effectively maintained.

FIG. 4C is a schematic view of an area through which the current flowing to the skin flows when the substantially L-shaped biogalvanic battery therapeutic appliance is brought into contact with the skin. The advantage of the biogalvanic battery therapeutic appliance being L-shaped, as can be seen in FIG. 4C, is that it is possible to form a wide-range ion current band surrounded by the L-shaped biogalvanic battery therapeutic appliance.

(Fourth example)

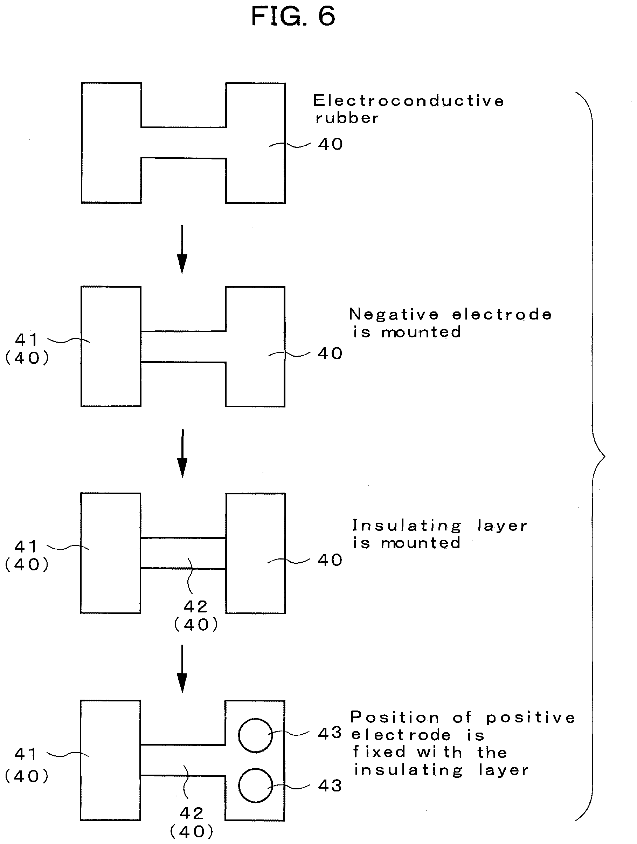

FIG. 6 is an explanatory drawing of the steps of manufacturing an H-shaped biogalvanic battery therapeutic appliance.

The following is described in the order of the manufacturing steps. An electroconductive member and H-shaped electroconductive rubber 40 constituting a positive electrode constituent member are prepared.

Next, a negative electrode constituent member 41 is mounted on one piece of the H-shaped electroconductive rubber 40 (rising piece on the left side of the diagram).

Next, an insulating layer 42 is mounted on the lateral piece of the H-shaped electroconductive rubber 40.

Then, an insulating layer 42 is additionally mounted on the other piece of the H-shaped electroconductive rubber 40 (rising piece on the right side of the diagram), elsewhere beside the locations of the electroconductive rubber 40 that are to come into contact with the skin and function as a positive electrode constituent member 43 (the two circular areas in the diagram).

Through these steps, an H-shaped biogalvanic battery therapeutic appliance according to the present invention is obtained.

In this biogalvanic battery therapeutic appliance, the near side of the plane of the diagram is the surface that comes into contact with the skin, and the location of the electroconductive rubber 40 covered by the insulating layer 42 and the positive electrode constituent member 43 functions as an electroconductive member, and the two circular areas not covered by the insulating layer 42 function as the positive electrode constituent member 43.

In FIG. 6, the reference numerals in parentheses indicate the reference numerals of the members on the far side of the plane of the diagram.

(Fifth example)

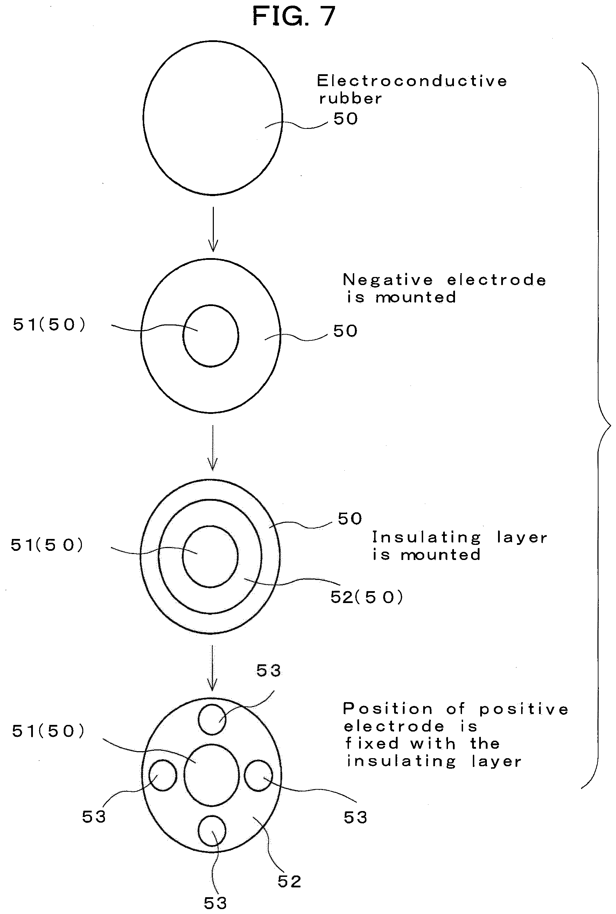

FIG. 7 is an explanatory drawing of the steps of manufacturing a disc-shaped biogalvanic battery therapeutic appliance.

The following is described in the order of the manufacturing steps. Circular electroconductive rubber 50 to constitute an electroconductive member and a positive electrode constituent member is prepared.

Next, a negative electrode constituent member 51 is mounted in the center of the electroconductive rubber 50.

Next, an insulating layer 52 is mounted on the outer periphery of the negative electrode constituent member 51.

Then, where the electroconductive rubber 50 is exposed, an insulating layer 52 is additionally mounted on the outer periphery of the insulating layer 52 elsewhere beside locations where the electroconductive rubber 50 is to come into contact with the skin and function as a positive electrode constituent member 53 (the four circular areas in the drawing).

Through these steps, a disc-shaped biogalvanic battery therapeutic appliance according to the present invention is obtained.

In this biogalvanic battery therapeutic appliance, the near side of the plane of the diagram is the surface that comes into contact with the skin, and of the electroconductive rubber 50, the location covered by the insulating layer 52 and the positive electrode constituent member 53 functions as an electroconductive member, and the four circular areas not covered by the insulating layer 52 function as the positive electrode constituent member 43.

In FIG. 6, the reference numerals in parentheses indicate the reference numerals of the members on the far side of the plane of the diagram.

In the examples described above, flat-plate-form, substantially L-shaped, H-shaped, and disc-shaped biogalvanic battery therapeutic appliances were described, but the present invention is not specified to biogalvanic battery therapeutic appliances of these shapes; the present invention includes biogalvanic battery therapeutic appliances of various shapes depending on, *inter alia,* the intended use and the location of application.

The negative electrode constituent member has a standard unipolar potential lower than that of the positive electrode component of the positive electrode constituent member, but metallic zinc is particularly suitable as the negative electrode component.

In addition, the positive electrode component constituting the positive electrode constituent member may be a metal having an electrode potential higher than that of the negative electrode component; for example, the positive electrode component may be gold (Au), silver (Ag), a platinum group, alloy thereof, etc. There are no particular limitations as to the grain size of the noble metal fine grains, but from the viewpoint of constituting numerous biogalvanic battery units, a grain size of finer grains is preferred, while from the viewpoint of manufacturing, coarse grains are easier to handle. If a compromise is made between these two viewpoints, it is possible to use noble metal fine grains having, for example, an average grain size of 1 nm to 50 $\mu$m, an average grain size of 20 nm to 15 $\mu$m, an average grain size of 10 to 15 $\mu$m, an average grain size of 20 to 40 nm, etc. However, fine grains of such description are not provided by way of limitation on the present invention.

In the above examples, carbon is given as an example of an electroconductive member, but examples of materials other than carbon include graphite, salt contents, electroconductive polymer materials or electroconductive polymers, etc. It is suitable to use a gel-form electroconductive polymer because such a polymer inherently has an adhesive effect and it is therefore not necessary to blend a binder, an adhesive, etc., therewith. Examples of typical substances for electroconductive polymer materials include polyacetylene, polyaniline, poly(p-phenylene vinylene), polypyrrole, polythiophene, polyaniline, poly(p-phenylene sulfide), polyethylene dioxythiophene (PEDOT), etc. Other examples are oligothiophene, etc. The actual properties are in some cases more like properties of semiconductors than of conductors. In addition, "ITO" processed from metal can also be used.

However, these electroconductive materials are processed, synthesized, or combined, and cannot be said to be inexpensive and stable. From such a viewpoint, carbon (including carbon nanotubes) is the least expensive and most stable, and can be said to be a safe material for the human body.

INDUSTRIAL APPLICABILITY

This invention inexpensively provides a biogalvanic battery therapeutic appliance that, despite the appliance having a high electrical resistance value and a long negative electrode constituent member, makes it possible to cause generated ions to efficiently flow to the negative electrode, and an excellent current stimulation effect of conductivity along the length of the negative electrode is obtained.

Specifically, the present invention functions particularly effectively in cases such as when the electrical resistance value is about 1 to 50 $\Omega \cdot$cm, the length of the negative electrode constituent member is 20 to 50 mm, the thickness of the negative electrode constituent member is 150 $\mu$m or less, and the "negative electrode constituent member length / negative electrode constituent member thickness" ratio is 1 or greater and preferably 100 or greater.

As a result, this biogalvanic battery therapeutic appliance, due to promoting blood circulation and purifying locally accumulated waste products, can be effectively used in the fields of beauty such as treatment and prevention of ailments such as stiff shoulders and lower-back pain, maintenance of beautiful skin, and improvement of skin quality.

Key

> 11, 21, 31, 41, 51: negative electrode constituent member, zinc silicon
> 12, 22, 32, 43, 53: positive electrode constituent member, noble metal silicon
> 13, 23, 33: electroconductive member, carbon silicon sheet
> 24, 34 (34a, 34b), 35, 42, 52: insulating layer, normal silicon, first and second insulating layers
> 40, 50: electroconductive rubber

**Claims**

1. A biogalvanic battery therapeutic appliance comprising a negative electrode constituent member, a positive electrode constituent member, and an electroconductive member disposed connected between the negative electrode constituent member and the positive electrode constituent member, electric circuits being formed between a living subject and the negative electrode constituent member and between the living subject and the positive electrode constituent member by bringing the negative electrode constituent member and the positive electrode constituent member into contact with the living subject, wherein

   > the negative electrode constituent member and the positive electrode constituent member each have a skin-contacting surface that comes into contact with the skin and an opposing surface formed on a surface that opposes the skin-contacting surface,
   > the negative electrode constituent member and the positive electrode constituent member are electrically connected by bridging and connecting the electroconductive member between the opposing surface of the negative electrode constituent member and the opposing surface of the positive electrode constituent member, and
   > the biogalvanic battery therapeutic appliance is **characterized in that** the electroconductive member and the positive electrode constituent member are constituted of same carbon material.

2. The biogalvanic battery therapeutic appliance according to claim 1, wherein the electroconductive member bridged and connected between the opposing surface of the negative electrode constituent member and the opposing surface of the positive electrode constituent member is disposed on the surface side opposite to the skin-contacting surface of the negative electrode constituent member, from a starting point that is the location where the distance from the positive electrode constituent member is the largest, to the positive electrode constituent member.

3. The biogalvanic battery therapeutic appliance according to claim 1, wherein the electroconductive member is formed coated on at least the entire surface of the skin-contacting surface side of the negative electrode constituent member.

4. The biogalvanic battery therapeutic appliance according to any of claims 1 to 3, wherein the negative electrode constituent member and the positive electrode constituent member are disposed separate from and opposing each other.

5. The biogalvanic battery therapeutic appliance according to any of claims 1 to 3, wherein the negative electrode constituent member and the positive electrode constituent member are disposed in contact with each other.

6. The biogalvanic battery therapeutic appliance according to any of claims 1 to 5, wherein the electroconductive member has an electrically insulating layer formed coated on the top surface of the surface opposite to the skin elsewhere beside the area that comes into contact with the positive electrode constituent member and the negative electrode constituent member, and contact between the electroconductive member and the skin is blocked by the electrically insulating layer.

7. The biogalvanic battery therapeutic appliance according to any of claims 1 to 6, wherein the negative electrode constituent member has a length of 10 to 200 mm, a thickness of 150 $\mu$m or less, and a "negative electrode constituent member length / negative electrode constituent member thickness" ratio of 1 or greater.

**8.** The biogalvanic battery therapeutic appliance according to claim 7, wherein the negative electrode constituent member has a "negative electrode constituent member length / negative electrode constituent member thickness" ratio of 100 or greater.

# FIG. 1

1 (Skin-contacting surface) 3 2

(Surface side that is opposite
to the skin-contacting surface)

# FIG. 2A

11                    (Skin-contacting surface)                    12

(Surface side that is opposite
to the skin-contacting surface)

13

# FIG. 2B

11                    (Skin-contacting surface)                    12

(Surface side that is opposite
to the skin-contacting surface)

13

# FIG.3

23     (Carbon silicon)

21     (Skin side surface)

23     $Ca\left(\begin{array}{c}\text{rbon silicon} \\ + \\ \text{Zinc silicon}\end{array}\right)$

24     (Surface that is opposite to the skin side surface)

$\left(\begin{array}{c}\text{Carbonsilicon} \\ + \\ \text{Regular silicon}\end{array}\right)$

22     (Skin side surface)

21     $\left(\begin{array}{c}\text{Carbon silicon} \\ + \\ \text{Zinc silicon} \\ + \\ \text{Noble metal silicon}\end{array}\right)$

# FIG. 4A

32

35

42

Surface that will contact
with the skin

41

31

# FIG. 4B

32

34b

42

Surface that will contact
with the skin

43

41

31

# FIG. 4C

32

34b

42

Ion
current
band

41      31

EP 4 094 801 A1

# FIG. 5

# FIG. 6

Electroconductive
rubber

~40

↓

Negative electrode
is mounted

41
(40)

~40

↓

Insulating layer
is mounted

41
(40)

~40

42
(40)

↓

Position of positive
~43 electrode is
fixed with the
insulating layer

41
(40)

~43

42
(40)

# FIG. 7

Electroconductive
rubber
50

Negative electrode
is mounted

51(50)    50

50

Insulating layer
is mounted

51(50)

52(50)

53

Position of positive
electrode is
fixed with the
insulating layer

51(50)

53    53

52

53

**EP 4 094 801 A1**

<table>
<tr><td colspan="2" align="center"><strong>INTERNATIONAL SEARCH REPORT</strong></td><td>International application No.<br>PCT/JP2021/002059</td></tr>
</table>

**A. CLASSIFICATION OF SUBJECT MATTER**

A61N 1/20(2006.01)i
FI: A61N1/20

According to International Patent Classification (IPC) or to both national classification and IPC

**B. FIELDS SEARCHED**

Minimum documentation searched (classification system followed by classification symbols)
A61N1/20

Documentation searched other than minimum documentation to the extent that such documents are included in the fields searched

| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | WO 2018/194079 A1 (NIPPON TELEGRAPH AND TELEPHONE CORP.) 25 October 2018 (2018-10-25) paragraphs [0001], [0033], [0038], [0042], [0045], fig. 4, 19 | 1–4<br>6–8<br>5 |
| Y | WO 2019/146714 A1 (KABUSHIKI KAISYA LEBEN) 01 August 2019 (2019-08-01) paragraphs [0046], [0051], fig. 1, 2 | 6–8 |

☐ Further documents are listed in the continuation of Box C. ☒ See patent family annex.

| | | |
|---|---|---|
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 19 February 2021 (19.02.2021) | 23 March 2021 (23.03.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

Information on patent family members

International application No.

PCT/JP2021/002059

| Patent Documents referred in the Report | Publication Date | Patent Family | Publication Date |
|---|---|---|---|
| WO 2018/194079 A1 | 25 Oct. 2018 | CN 110418662 A paragraphs [0002], [0068], [0074], [0079], [0084], fig. 4, 19 | |
| WO 2019/146714 A1 | 01 Aug. 2019 | CN 110612141 A paragraphs [0097], [0102], fig. 1, 2 | |

Form PCT/ISA/210 (patent family annex) (January 2015)

**REFERENCES CITED IN THE DESCRIPTION**

**Patent documents cited in the description**

- JP 6168639 B
- JP 6153259 B